Europäisches Patentamt

European Patent Office

Office européen des brevets

(19)

(11) Numéro de publication: **0 327 468**
**A1**

## DEMANDE DE BREVET EUROPEEN

(12)

(21) Numéro de dépôt: 89400318.5

(22) Date de dépôt: 03.02.89

(51) Int. Cl.⁴: **A 61 F 5/453**

(30) Priorité: 05.02.88 FR 8801360

(43) Date de publication de la demande:
09.08.89 Bulletin 89/32

(84) Etats contractants désignés:
AT BE CH DE ES FR GB GR IT LI LU NL SE

(71) Demandeur: CENTRE NATIONAL D'ETUDES
SPATIALES
2 Place Maurice-Quentin
F-75039 Paris Cedex 01 (FR)

COMAT S.à.r.l.
ZI Vidailhan 5 rue Vidailhan
F-31130 Balma (FR)

(72) Inventeur: **Raffin, Jacques**
**5 allée des Félibres**
**F-31520 Ramonville St Agne (FR)**

**Cunisse, Michel**
**11 rue Watteau**
**F-31240 Saint Jean (FR)**

**Peyre, Guy**
**Gensac sur Garonne**
**F-31310 Montesquieu-Volvestre (FR)**

(74) Mandataire: **Mongrédien, André et al**
**c/o BREVATOME 25, rue de Ponthieu**
**F-75008 Paris (FR)**

(54) Appareil pour recueillir l'urine rejetée naturellement par l'homme.

(57) Appareil pour recueillir l'urine rejetée naturellement par l'homme.

Il comprend un réservoir (1) à clapet (12) verrouillable et un ensemble tubulaire (2, 3) dans lequel l'homme introduit son pénis. Un manchon interne de latex (41) procure l'imperméabilité en amont. Un fourreau absorbant (35) recueille l'urine n'ayant pas pénétré dans le réservoir.

Application notamment dans les véhicules spatiaux en condition d'apesanteur ou en milieu médical.

FIG. 1

EP 0 327 468 A1

Description

## APPAREIL POUR RECUEILLIR L'URINE REJETEE NATURELLEMENT PAR L'HOMME

La présente invention a pour objet un appareil pour recueillir l'urine rejetée par voie naturelle par l'homme, ou plus précisément par l'individu de sexe masculin.

Elle peut être utilisée dans tous les milieux confinés dépourvus d'installations sanitaires et dont le caractère clos nécessite une hygiène d'autant plus rigoureuse. Il peut s'agir en particulier de véhicules sous-marins ou spatiaux. En particulier, la réalisation plus précisément décrite ci-après a été étudiée pour fonctionner de façon satisfaisante en apesanteur. Il est en effet absolument nécessaire dans ce cas d'éviter de laisser des gouttelettes d'urine se déposer sur les parois de l'appareil, qui viendraient ensuite polluer l'atmosphère.

L'invention est également utile pour des handicapés ne pouvant accéder à une installation sanitaire normale et peut donc en particulier s'envisager dans des hôpitaux et cliniques.

Plus précisément, l'invention a pour objet un appareil destiné à recueillir l'urine rejetée naturellement par l'homme, caractérisé en ce qu'il comprend un ensemble tubulaire par une première extrémité duquel l'homme introduit le pénis, un élément lié à l'ensemble tubulaire se refermant autour du pénis et s'en écartant tel qu'un manchon élastique, un réservoir délimitant un volume interne, le réservoir étant muni d'une ouverture et lié à la seconde extrémité de l'ensemble tubulaire par un raccordement étanche disposé autour de l'ouverture, et un clapet obstruant l'ouverture en l'absence de rejet d'urine et l'ouvrant quand l'urine est rejetée.

Avantageusement, l'ensemble tubulaire comprend à sa seconde extrémité un fourreau tubulaire absorbant s'étendant autour de l'ouverture du réservoir et du clapet.

Selon une caractéristique avantageuse, le réservoir comprend une portion en caoutchouc perçable par une aiguille de seringue et pouvant se refermer sur le perçage après retrait de l'aiguille, ainsi qu'une autre portion résistant aux piqûres de l'aiguille et disposée en face de la portion de caoutchouc.

Des systèmes de verrouillage contrarient avantageusement la désolidarisation des pièces constituant l'appareil. Par ailleurs, dans la réalisation préférée, le manchon élastique et une paroi rigide de l'ensemble tubulaire autour du manchon délimitent un volume annulaire étanche qui communique avec une poire d'aspiration de l'air contenu dans le volume annulaire, un moyen étant par ailleurs prévu pour faire communiquer le volume annulaire avec l'extérieur. De manière optimale, ce même moyen verrouille généralement le clapet en position de fermeture et fait communiquer le volume annulaire avec l'extérieur dès que le clapet est déverrouillé.

On va maintenant décrire plus précisément l'invention à l'aide des figures suivantes, annexées à titre illustratif et nullement limitatif :

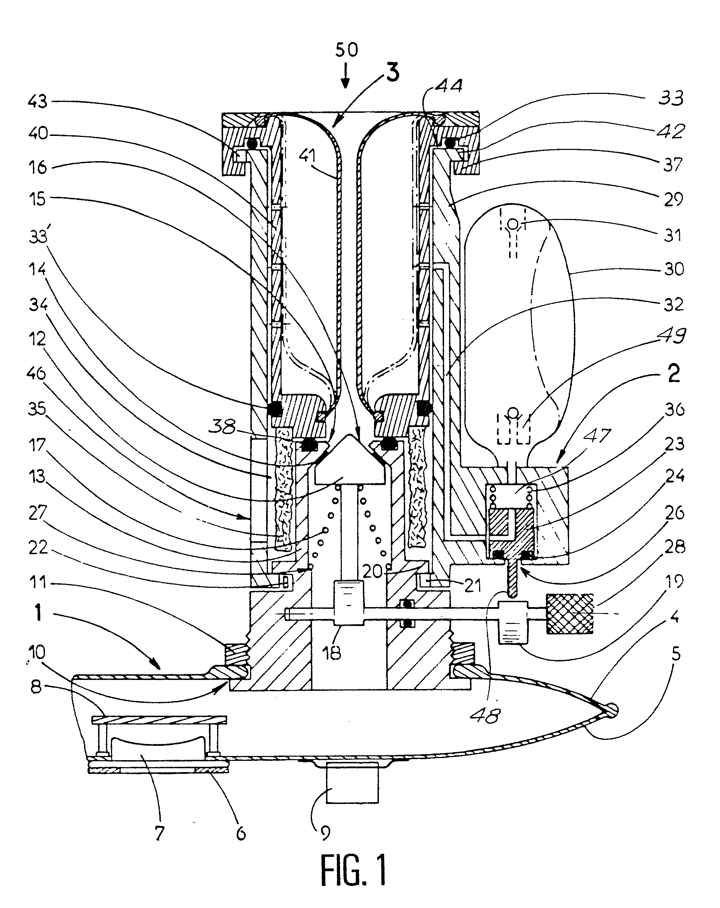

- la figure 1 représente la majeure partie de l'invention, et

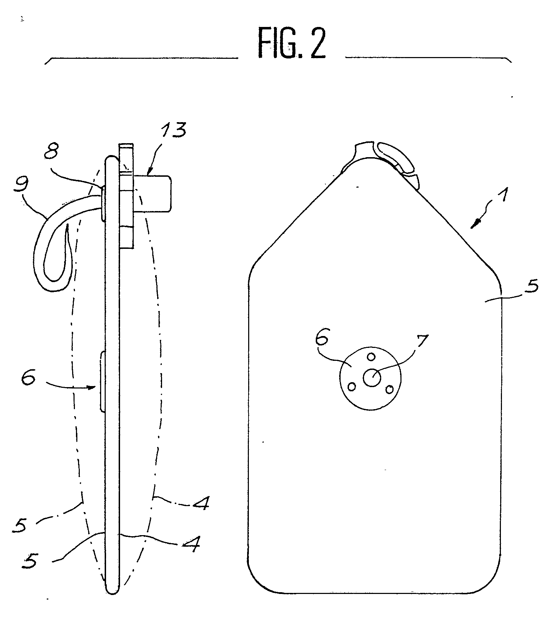

- la figure 2 représente le réservoir.

Les figures montrent que la réalisation décrite comprend essentiellement trois ensembles : un réservoir 1, un corps pneumatique 2 et un étui 3. Le réservoir 1 se compose tout d'abord de deux parois 4 et 5 planes au repos et élastiques assemblées à leurs contours : le réservoir 1 vide est plat et bombé quand il est plein. Une paroi 5 est percée d'un orifice autour duquel est établie une bride 6 qui tend un bouchon plat de caoutchouc 7. Ce bouchon 7 peut être percé par l'aiguille d'une seringue pour aspirer le contenu du réservoir 1 ; la piqûre de l'aiguille est automatiquement colmatée après que cette dernière a été extraite grâce à l'expansion du caoutchouc l'étanchéité est maintenue. Une plaque métallique 8 est établie derrière la paroi 5 en face du bouchon 7 pour éviter que l'aiguille de la seringue perce cette paroi 4 opposée. Une poignée 9 est fixée sur la paroi 5 et permet la manutention du réservoir 1.

La liaison du réservoir 1 avec les autres pièces de l'invention s'effectue à l'aide d'un raccordement 10 établi dans un orifice de la paroi 4, maintenu par une collerette 11 de serrage et prolongé hors du réservoir 1 par un tube 13 dans lequel se déplace axialement un clapet 12. Le tube 13 comprend à son extrémité extérieure un épaulement 14 qui empêche le clapet 12 de sortir et comprend une surface extérieure conique 15 convergeant vers le réservoir 1. Le clapet 12 comprend une surface extérieure 16 tronconique s'effilant en s'éloignant du réservoir 1 et dont une grande partie est plus fine que le diamètre intérieur de l'épaulement 14 et peut donc dépasser de cet épaulement. Un ressort de compression 17 de faible raideur dans le tube 13 et s'appuyant sur une butée interne 27 permet en temps normal de plaquer le clapet 12 contre l'épaulement 14.

Un robinet 28 est établi sur le tube 13 et permet de faire tourner deux cames : l'une 18 est interne et une de ses positions permet de bloquer le clapet 12 contre l'épaulement 14, une autre came 19, externe, sera décrite davantage plus loin. Une partie du tube 13 comprend des tenons 20 qui permettent d'y raccorder le corps pneumatique 2. Ce dernier comprend d'abord une paroi tubulaire 29 rigide pourvue à une extrémité de gorges 21 dans lesquelles on peut glisser les tenons 20 par un mouvement de baïonnette. Un repère de détrompage non représenté et pouvant consister en des graduations est établi sur les surfaces externes du tube 13 et du corps pneumatique 2 en regard. Les gorges 21 son en cul de sac et, près de leur extrémité, une lame élastique 22 que le tenon correspondant 20 peut écarter permet de verrouiller le corps pneumatique 2 sur le réservoir 1 à condition d'exercer un effort suffisant ; le tenon 20 est alors situé entre la lame 22 et le fond de la gorge 21. Le déverrouillage s'effectue par un mouvement inverse.

Un piston 23 est établi dans un évidement 47 du corps pneumatique 2 près du robinet 28. Il est prolongé par un doigt 48 pointant vers la came externe 19 à travers un passage 26 qui fait communiquer l'évidement 47 avec l'extérieur. Un ressort 36 plaque le piston 23 contre le passage 26, qui est isolé de l'évidement 47 par un joint d'étanchéité 24.

Quand le robinet 28 est tourné, la came externe 19 repousse le doigt 48 et établit la communication entre l'évidement 47 et l'extérieur.

Une poire d'aspiration 30 est fixée sur la paroi tubulaire 29 du corps pneumatique 2. Elle comprend une valve extérieure 31 et une valve intérieure 49 ; un conduit d'aspiration 32 la relie à l'intérieur de la paroi tubulaire 29 par l'intermédiaire de l'évidement 47 ; des pressions successives sur la poire d'aspiration 30 permettent d'évacuer l'air qu'elle contient par la valve extérieure 31 tout en aspirant l'air provenant de l'intérieur de la paroi tubulaire 29 par le conduit d'aspiration 32. La valve intérieure 49 empêche les retours d'air vers le conduit d'aspiration 32. Dans la réalisation représentée, le piston 23 est situé devant l'ouverture du conduit d'aspiration 32 et est donc muni d'un conduit de prolongement coudé reliant le conduit d'aspiration 32 à l'évidement 47.

Le corps pneumatique 2 comprend enfin des gorges 43 à l'extrémité de raccordement 42 opposée au tube 13, par laquelle on racccorde l'étui 3.

L'étui 3 comprend une paroi ajourée tubulaire 40 rigide, un manchon élastique de latex 41 à l'intérieur, des tenons de raccordement 37 et un fourreau absorbant 35. La paroi ajourée 40 est introduite au cours du montage dans la paroi tubulaire 29 par translation axiale, glisse par un joint torique 33′ et s'appuie sur un autre joint torique 33. Un autre joint torique 38 entre les faces planes d'extrémité du tube 13 et de l'étui 3 assure l'étanchéité avec l'extérieur à cet endroit. On fait ensuite pénétrer les tenons 37 de l'étui 3 dans les gorges 43 établies dans l'extrémité de raccordement 42 pour réaliser un blocage en rotation. Un léger verrouillage est obtenu en faisant frotter une portée 44 de l'extrémité de raccordement 42 contre le joint torique 33.

Le fourreau absorbant 35 est maintenu par une extrémité de la paroi ajourée 40 opposée à l'extrémité de raccordement 42 en dépassant de cette paroi. Il s'étend dans un logement 34 de forme annulaire délimité par le tube 13 à l'intérieur et la paroi tubulaire 29 à l'extérieur. Une découpe 46 ménagée dans cette dernière permet d'accéder au fourreau absorbant 35, qui peut être renforcé par une bande souple imperméable cylindrique qui l'entoure.

Quand l'assemblage du réservoir 1, du corps pneumatique 2 et de l'étui 3 a été effectué, on peut procéder au fonctionnement de l'appareil. A cet instant, la came interne 18 bloque le clapet 12 contre l'épaulement 14. La came externe 19 n'est pas en contact avec le doigt 48 et le manchon de latex 41 obstrue presque complètement l'étui 3 par suite d'un gondolage naturel.

Des pressions manuelles répétées sur la poire d'aspiration 30 aspirent l'air contenu dans le volume délimité par la paroi tubulaire 29 et le manchon de latex 41 (et que la paroi ajourée 40 ne permet pas de fractionner) si bien que ce dernier 41 est alors plaqué contre la paroi ajourée 40 suivant la position indiquée en tirets. L'intérieur de l'étui 3 est alors dégagé et l'utilisateur peut y introduire son pénis suivant la flèche 50. Ensuite, l'utilisateur ouvre le robinet 28 : la came interne 18 débloque le clapet 12 et la came externe 19 fait remonter le piston 23 qui dégage alors le passage d'amenée d'air 26. L'air extérieur pénètre alors dans le conduit d'aspiration 32, si bien que le manchon de latex 41 reprend sa forme initiale et se referme autour du pénis qu'il maintient désormais en place. Il est alors possible d'uriner dans les conditions d'hygiène souhaitées. La pression du liquide repousse le clapet 12 et l'urine pénètre dans le réservoir 1. Quand l'évacuation est achevée, le clapet 12 se remet en place sous la pression du ressort de compression 17.

L'emploi de la pression ambiante pour obtenir la fermeture étanche du manchon de latex 41 est beaucoup plus sûr que si une surpression devait être créée et correspond donc à un avantage sensible de cette réalisation, de même que l'établissement automatique de la pression ambiante lors de l'ouverture du clapet 12.

L'utilisateur referme ensuite le robinet 28, bloquant le clapet 12 et obturant le passage 26 par le joint 24 du piston 23, ce dernier étant naturellement ramené par le ressort 36. L'utilisateur peut ensuite agir sur la poire d'aspiration 30 pour dégager le pénis du manchon de latex 41.

Lors du désaccouplement des parties 1, 2 et 3 le fourreau absorbant 35 peut être saisi à travers la découpe 46 et permet d'essuyer les gouttes d'urine résiduelles.

Le réservoir 1 peut être détaché, jeté et remplacé quand il est plein. Il peut par ailleurs permettre un prélèvement à l'aide d'une seringue dont l'aiguille vient percer le bouchon 7. On évite par ce moyen des vidanges accidentelles qui seraient très gênantes en environnement confiné. Si on utilise le dispositif en milieu hospitalier, on peut cependant vouloir utiliser plusieurs fois le réservoir 1 que l'on munira alors d'un robinet de vidange quelconque.

**Revendications**

1. Appareil pour recueillir l'urine rejetée naturellement par l'homme, caractérisé en ce qu'il comprend un ensemble tubulaire (2, 3) par une première extrémité duquel l'homme introduit le pénis, un élément (41) lié à l'ensemble tubulaire se refermant autour du pénis et s'en écartant, un réservoir (1) délimitant un volume interne, le réservoir étant muni d'une ouverture et lié à la seconde extrémité de l'ensemble tubulaire par une pièce de raccordement (13) étanche comprenant l'ouverture cylindrique, et un clapet (12) obstruant cette ouverture en l'absence de rejet d'urine et l'ouvrant quand l'urine est rejetée.

2. Appareil selon la revendication 1, caractérisé en ce que l'ensemble tubulaire (2, 3) comprend à sa seconde extrémité un fourreau tubulaire (35) absorbant et s'étendant autour de l'ouverture du réservoir et du clapet (12).

3. Appareil selon l'une quelconque des revendications 1 ou 2, caractérisé en ce que l'élément se refermant et s'écartant est un manchon élastique (41) tapissant l'intérieur de l'ensemble tubulaire (2, 3) vers sa première extrémité.

4. Appareil selon la revendication 3, caractérisé en ce que l'ensemble tubulaire (2, 3) comprend une paroi (29) autour du manchon (41) et délimitant un volume annulaire étanche avec le manchon (41), une poire (30) aspirant l'air contenu dans le volume annulaire et un moyen (23, 24, 26, 36) pour faire communiquer le volume annulaire avec l'extérieur.

5. Appareil selon la revendication 4, caractérisé en ce que l'ensemble tubulaire comprend un corps (2) auquel appartient la paroi (29) et un étui (3) auquel appartient le manchon (41), l'étui (3) étant concentrique dans le corps (2) et muni d'une paroi perforée (40) rigide entre la paroi (29) et le manchon (41).

6. Appareil selon l'une quelconque des revendications 4 ou 5, caractérisé en ce que le manchon (41) présente, en l'absence d'aspiration, un gondolage central s'étendant radialement vers l'intérieur.

7. Appareil selon les revendications 2 et 5, caractérisé en ce que le corps (2) entoure le fourreau absorbant (35) et présente une découpe (46) en face du fourreau absorbant (35).

8. Appareil selon l'une quelconque des revendications 1 à 7, caractérisé en ce que l'ouverture est en entonnoir (15) s'élargissant vers l'étui et en ce que le clapet (12) présente un bombement conique (16) s'amincissant vers l'ensemble tubulaire (2, 3) et placé dans l'entonnoir (16) quand l'ouverture est obstruée.

9. Appareil selon l'une quelconque des revendications 1 à 8, caractérisé en ce que le réservoir (1) est souple.

10. Appareil selon l'une quelconque des revendications 1 à 9, caractérisé en ce que le réservoir (1) comprend une portion en caoutchouc (7) perçable par une aiguille de seringue et pouvant se refermer sur le perçage après retrait de l'aiguille, ainsi qu'une autre portion (8) résistant aux piqûres de l'aiguille et disposée en face de la portion en caoutchouc (7).

11. Appareil selon l'une quelconque des revendications 5 à 10, caractérisé en ce que l'étui (3) et le corps (2) sont séparables.

12. Appareil selon l'une quelconque des revendications 1 à 11, caractérisé en ce que le réservoir (1) et l'ensemble tubulaire (2, 3) sont séparables.

13. Appareil selon l'une quelconque des revendications 1 à 12, caractérisé en ce que le clapet (12) est verrouillable dans une position où il obstrue l'ouverture du réservoir.

14. Appareil selon les revendications 4 et 13, caractérisé en ce que l'interruption du verrouillage du clapet (12) fait communiquer le volume annulaire avec l'extérieur.

FIG. 1

# FIG. 2

Office européen
des brevets

RAPPORT DE RECHERCHE EUROPEENNE

Numero de la demande

EP 89 40 0318

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 239 044  (PAVLINCH)<br>* figure 3, revendication 1 *<br>--- | 1,3 | A 61 F   5/453 |
| A | US-A-3 526 227  (APPELBAUM)<br>* figure 1; colonne 2, lignes 14-29 *<br>--- | 1 | |
| A | WO-A-8 201 648  (HAAN)<br>* figure 1 *<br>--- | 1 | |
| A | US-A-3 511 241  (LEE)<br>* figures 1,10 *<br>----- | 1,3 | |

DOMAINES TECHNIQUES RECHERCHES (Int. Cl.4)

A 61 F   5/00

Le présent rapport a été établi pour toutes les revendications

| Lieu de la recherche | Date d'achèvement de la recherche | Examinateur |
|---|---|---|
| BERLIN | 25-04-1989 | KANAL P K |

EPO FORM 1503 03.82 (P0402)